(19) Europäisches Patentamt
European Patent Office
Office européen des brevets

(11) Veröffentlichungsnummer: **0 011 773**
**B1**

(12) # EUROPÄISCHE PATENTSCHRIFT

(45) Veröffentlichungstag der Patentschrift: **28.10.81**

(51) Int. Cl.³: **C 07 C 143/86**

(21) Anmeldenummer: **79104496.9**

(22) Anmeldetag: **14.11.79**

(54) **Verfahren zur Herstellung von Amidosulfonsäuren.**

(30) Priorität: **02.12.78 DE 2852159**

(43) Veröffentlichungstag der Anmeldung: **11.06.80 Patentblatt 80/12**

(45) Bekanntmachung des Hinweises auf die Patenterteilung: **28.10.81 Patentblatt 81/43**

(84) Benannte Vertragsstaaten: **BE CH DE FR GB IT NL**

(56) Entgegenhaltungen: **DE-A-2 424 371**

(73) Patentinhaber: **BASF Aktiengesellschaft, Carl-Bosch-Strasse 38, D-6700 Ludwigshafen (DE)**

(72) Erfinder: **Jacobs, Peter, Dr. Dipl.-Chem., Hanns-Fay-Strasse 4, D-6710 Frankenthal (DE)**
Erfinder: **Mangold, Dietrich, Dr. Dipl.-Chem., Hermann-Walker-Strasse 49, D-6903 Neckargemuend (DE)**
Erfinder: **Hamprecht, Gerhard, Dr. Dipl.-Chem., Rote-Turm-Strasse 28, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 773 B1

ACTORUM AG.

# Verfahren zur Herstellung von Amidosulfonsäuren

Die Erfindung betrifft ein neues Verfahren zur Herstellung von Amidosulfonsäuren durch Umsetzung von Harnstoffen mit Oleum bestimmter Zusammensetzung zuerst bei tiefer und dann bei höherer Temperatur in Gegenwart organischer Lösungsmittel.

Es ist aus Houben-Weyl, Methoden der Organischen Chemie, Band 11/2, Seiten 654 und 655, bekannt, dass N,N'-Dialkylharnstoffe durch Einwirkung von Oleum zuerst sulfiert und dann zu Amidosulfonsäuren gespalten werden. Es wird darauf hingewiesen, dass die Harnstoffspaltung bei nur wenig höherer Temperatur als die Sulfierung eintritt, und gefordert, dass man unter möglichst milden Reaktionsbedingungen arbeitet, um eine Spaltung von z.B. Alkylharnstoff vor der Sulfierung und damit verbunden eine Bildung von z.B. saurem Alkylammoniumsulfat zu vermeiden. Wie das angegebene Beispiel der Herstellung von Methylsulfamidsäure zeigt, liegt die Reaktionstemperatur bei 0 bis höchstens 45 °C. Organische Lösungsmittel werden der Reaktion nicht zugesetzt, 30prozentiges Oleum wird in grossem Überschuss eingesetzt. Zur Isolierung der reinen Säure sind umständliche Reinigungsoperationen mit Äther erforderlich.

Das deutsche Reichspatent 636 329 zeigt dieselbe Umsetzung mit 35prozentigem Oleum und erwähnt eine Nachbehandlung mit Schwefelsäure oder Wasser, ohne diese Ausführungsformen näher zu beschreiben. Zwar wird erwähnt, dass die Reaktion durch Erwärmen auf dem Wasserbad zu Ende geführt werden kann, jedoch wird weder ein 2-Stufen-Verfahren mit verschiedenen Temperaturintervallen beschrieben, noch überhaupt als entscheidende Massnahme erkannt. Organische Lösungsmittel werden nicht verwendet.

Eine Arbeit im Journal of the American Chemical Society, Band 75, Seite 1 408 (1953) zeigt ebenfalls die von Houben-Weyl beschriebene Umsetzung mit Oleum ohne weitere Nachbehandlung; es wird ausdrücklich auf die Schwierigkeit hingewiesen, bei der Reaktion ein Optimum an Ausbeute zu erzielen und insbesondere die Bildung von Alkylammoniumsulfat zu unterdrücken oder zu verringern. Die Zeit der Zugabe der Ausgangsstoffe und die Temperaturregelung der Reaktion spielen eine erhebliche Rolle. Bei der Aufarbeitung muss der Endstoff mehrfach mit Äther gewaschen werden, enthält aber trotz dieser Reinigungsoperation noch Sulfat und kann nur durch Lösen in Methanol und Fällung durch Zugabe grösserer Mengen an Äther gereinigt werden. Als Reaktionstemperatur werden Temperaturen unter 45 °C beschrieben und darauf hingewiesen, dass bei ungenügender Kühlung – das Beispiel zeigt Eiskühlung – Reinheit und Ausbeute des Endstoffs erheblich abnehmen. Man verwendet lediglich 30prozentiges Oleum im Überschuss.

Die britische Patentschrift 1 185 439 weist auf den Nachteil hin, Schwefelsäure, auch in Gestalt von Oleum, zu verwenden, da man bei Verwendung von Schwefelsäure stets einen stark verunreinigten Endstoff erhält und die Entfernung der Schwefelsäure umständlich und schwierig ist. Sie empfiehlt daher eine Arbeitsweise, bei der substituierter Harnstoff mit wenigstens doppelten äquimolaren Mengen an Schwefeltrioxid in Gegenwart eines organischen Lösungsmittels umgesetzt wird.

Es wird betont, dass üblicherweise 2 bis 4, vorzugsweise 3 Mol Schwefeltrioxid je Mol Harnstoff angewendet werden. Die Umsetzung kann auch in zwei Stufen durchgeführt werden, wobei dem Reaktionsgemisch in jeder Stufe Schwefeltrioxid zugeführt werden muss. Die Ausbeuten sind unbefriedigend. Ein wesentlicher Nachteil dieser Verfahrensweise ist in der technisch sehr schwierigen Handhabung von Schwefeltrioxid zu sehen.

Die US-Patentschrift 3 555 081 beschreibt dieselbe Arbeitsweise bei der Synthese von N-Cyclohexylamidosulfonsäure und zeigt ebenfalls, dass die Verwendung von Schwefelsäure zu verunreinigten Endstoffen führt. Nach ihrer Lehre (Spalte 3, Zeilen 45 bis 54) ist es entscheidend, dass keine Schwefelsäure im Reaktionsgemisch anwesend ist. Bei zweistufigen Arbeitsweisen darf Schwefelsäure nur zusammen mit Schwefeltrioxid in Gestalt von Oleum im zweiten Reaktionsschritt verwendet werden.

Die deutsche Offenlegungsschrift 2 424 371 beschreibt eine zweistufige Verfahrensweise, in der man in einem organischen Lösungsmittel substituierte Harnstoffe in einem ersten Reaktionsschritt mit Schwefeltrioxid in einer Menge von 1 bis 1,9 Mol je Mol Ausgangsstoff umsetzt und das gebildete Reaktionsgemisch in einem zweiten Schritt mit Schwefelsäure in einer Menge von 1 bis 1,5 Mol je Mol Ausgangsstoff umsetzt. Ausdrücklich wird darauf hingewiesen, dass Stoffe, die Schwefelsäure enthalten, z.B. Oleum, nicht anstelle von Schwefeltrioxid in Betracht kommen. Ein wesentlicher Nachteil dieser Verfahrensweise ist in der technisch sehr schwierigen und aufwendigen Handhabung von Schwefeltrioxid zu sehen. Schwefeltrioxid muss im grosstechnischen Betrieb in der Regel durch Destillation in einer zusätzlichen Apparatur aus Oleum gewonnen oder mit Inertgasen wie Kohlendioxid oder Stickstoff langwierig aus Spezialgefässen ausgetrieben bzw. über beheizte Leitungen oder in teuren Spezialgefässen aus der Schwefelsäureproduktion bezogen werden. In der Veröffentlichung wird ausdrücklich gezeigt, dass die vorgenannten Verfahren, die von vornherein oder in der ersten Stufe Oleum verwenden, wesentlich schlechtere Ausbeute liefern.

Alle diese Verfahren sind mit Bezug auf optimale Ausbeute und Reinheit des Endstoffs bei gleichzeitig einfachem, betriebssicherem und wirtschaftlichem Betrieb, gerade im grosstechnischen Massstab, unbefriedigend.

Es wurde nun gefunden, dass man Amidosulfonsäuren der Formel

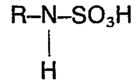

I,

worin R einen aliphatischen oder cycloaliphatischen Rest bedeutet, durch Umsetzung von Harnstoffen mit Oleum in Gegenwart organischer Lösungsmittel vorteilhaft erhält, wenn man substituierte Harnstoffe der Formel

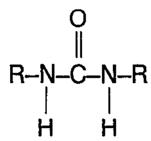

II,

worin R die vorgenannte Bedeutung hat, mit Oleum, das Schwefeltrioxid in einer Menge von 1 bis 2,5 Mol und Schwefelsäure in einer Menge von 1 bis 1,5 Mol je Mol Ausgangsstoff II enthält, in einem ersten Schritt bei einer Temperatur von –20 bis +50 °C und dann in einem zweiten Schritt bei einer Temperatur zwischen 50 und 140 °C umsetzt.

Die Umsetzung kann für den Fall der Verwendung von N,N'-Dimethylharnstoff durch die folgenden Formeln wiedergegeben werden:

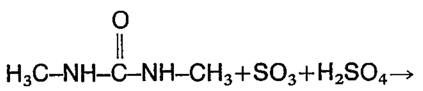

$2H_3C-NH-SO_3H+CO_2$.

Im Vergleich zu erstgenannten bekannten Verfahren liefert das Verfahren nach der Erfindung auf einfacherem und wirtschaftlicherem Wege Amidosulfonsäure in besserer Ausbeute und Reinheit. Es wird einbadig in zwei Stufen durchgeführt. Es ist ein entscheidendes Merkmal der Erfindung, dass in der zweiten Stufe weder Schwefeltrioxid noch Oleum zugeführt wird und lediglich Oleum in der ersten Stufe verwendet wird. Im Vergleich zu dem in der deutschen Offenlegungsschrift 2 424 371 beschriebenen Verfahren ist das erfindungsgemässe Verfahren einfacher, betriebssicherer und wirtschaftlicher; die Handhabung grösserer Mengen Schwefeltrioxid sowie der Betrieb entsprechender Spezialanlagen werden vermieden. Alle diese vorteilhaften Ergebnisse des erfindungsgemässen Verfahrens sind im Hinblick auf den Stand der Technik überraschend.

Bevorzugte Ausgangsstoffe II und dementsprechend bevorzugte Endstoffe I sind solche, in deren Formeln R einen Alkylrest mit 1 bis 12, vorzugsweise 1 bis 5 Kohlenstoffatomen oder einen Cyclohexylrest bedeutet. Die vorgenannten Reste können noch durch unter den Reaktionsbedingungen inerten Gruppen, z. B. Alkylgruppen mit 1 bis 4 Kohlenstoffatomen, substituiert sein.

Beispielsweise sind folgende Harnstoffe als Ausgangsstoffe II geeignet: N,N'-Dimethylharnstoff, N,N'-Diisopropylharnstoff, N,N'-Di-n-butylharnstoff, N,N'-Didodecylharnstoff, N,N'-Di-sek.-butylharnstoff, N,N'-Di-tert.-butylharnstoff, N,N'-Diäthylharnstoff, N,N'-Dicyclohexylharnstoff, N,N'-Di-n-propylharnstoff.

Die Umsetzung wird schon im ersten Schritt und somit bei der Gesamtreaktion mit 1 bis 2,5 Mol, vorzugsweise 1 bis 1,5 Mol, insbesondere 1 bis 1,1 Mol Schwefeltrioxid und mit 1 bis 1,5, vorzugsweise 1 bis 1,2, insbesondere 1 bis 1,1 Mol Schwefelsäure je Mol Ausgangsstoff II durchgeführt. Bevorzugt ist ein Verhältnis von ungefähr 1 Mol Schwefeltrioxid zu 1 Mol $H_2SO_4$, wobei die Abweichung von der Stöchiometrie vorteilhaft unter 10 Gewichtsprozent liegt. Insbesondere ist die Verwendung von 45gewichtsprozentigem Oleum (Pyroschwefelsäure $H_2S_2O_7$) in vorgenannten Mengenverhältnissen, insbesondere 178 bis 195 Gewichtsprozent Oleum bezogen auf 1 Mol Ausgangsstoff II, bevorzugt. Man kann anstelle von Oleum, zweckmässig 45gewichtsprozentigem Oleum, auch Gemische von höherkonzentriertem Oleum und/oder Schwefeltrioxid mit niederkonzentriertem Oleum, Schwefelsäure und/oder Wasser verwenden, wobei die Gemische auf ein Oleum der erfindungsgemässen Schwefeltrioxidmengen und Schwefelsäuremengen eingestellt werden. Schwefelsäure wird in der Regel für die Bildung des Oleums in Gestalt von Schwefelsäure (100%) (Monohydrat) verwendet; gegebenenfalls kommt auch 96- bis 100gewichtsprozentige, Wasser enthaltende Schwefelsäure für die Bildung des Oleums in Frage. Schwefeltrioxid kann für die Bildung des Oleums in fester oder zweckmässig in flüssiger Form oder als Gas zur Anwendung gelangen; vorteilhaft kommt für die Bildung des Oleums 100prozentiges Schwefeltrioxid in Frage, gegebenenfalls kann es auch mit Inertgas wie Kohlendioxid oder Stickstoff, verdünnt sein. Man kann aber auch Stoffe, die unter den Vermischungsbedingungen Schwefeltrioxid abgeben, für die Bildung des Oleums verwenden, beispielsweise Additionsverbindungen von Schwefeltrioxid, z. B. mit Äthern wie Tetrahydrofuran, Di-($\beta$-chloräthyl)-äther, 1,4-Dioxan; N,N-disubstituierten Carbonsäureamiden wie N,N-Dimethylformamid; mit tertiären Aminen, z. B. Pyridin, Triäthylamin, Trimethylamin, Tributylamin, Chinolin, Chinaldin, Dimethylanilin, Triphenylamin, N-Methylmorpholin, N-Äthylmorpholin, N-Methylpiperidin, N-Äthylimidazol, N-Methyläthylenimin, N-Äthylpentamethylenimin; oder Additionsverbindungen von Chlorsulfonsäure mit vorgenannten Aminen, insbesondere Pyridin; oder entsprechende Gemische. Bezüglich der Definition von 100prozentigem Schwefeltrioxid wird auf Ullmanns Encyklopädie der technischen Chemie, Band 15, Seiten 465 bis 467 und bezüglich der Herstellung von Additionsverbindungen auf Houben-Weyl, (loc. cit.) Band VI/2, Seiten 455 bis 457 und Band IX, Seiten 503 bis 508, verwiesen.

Die Umsetzung wird im ersten Schritt bei einer Temperatur von –20 bis +50 °C, zweckmässig von –10 bis +30 °C, vorzugsweise –5 bis +27 °C, im zweiten Schritt von oberhalb 50 °C bis unterhalb

140 °C, zweckmässig von 51 bis 100 °C, vorzugsweise 51 bis 85 °C, drucklos oder unter Druck, kontinuierlich oder diskontinuierlich durchgeführt. Man verwendet in beiden Schritten unter den Reaktionsbedingungen inerte organische Lösungsmittel, wobei vorteilhaft die Gesamtmenge an organischem Lösungsmittel schon dem ersten Reaktionsschritt zugegeben wird. Als Lösungsmittel kommen z. B. in Frage: Vorteilhaft Halogenkohlenwasserstoffe, insbesondere Chlorkohlenwasserstoffe, z. B. Tetrachloräthylen, Amylchlorid, Cyclohexylchlorid, Dichlorpropan, Methylenchlorid, Dichlorbutan, Chloroform, Tetrachorkohlenstoff, Tetrachloräthan, Trichloräthan, Trichloräthylen, Pentachloräthan, Trichlorfluormethan, cis-Dichloräthylen, 1,2-Dichloräthan, 1,1-Dichloräthan, n-Propylchlorid, 1,2-cis-Dichloräthylen, n-Butylchlorid, 2-, 3- und iso-Butylchlorid; und entsprechende Gemische. Zweckmässig verwendet man das Lösungsmittel in einer Menge von 200 bis 10 000 Gewichtsprozent, vorzugsweise von 300 bis 1000 Gewichtsprozent, bezogen auf Ausgangsstoff II.

Die Reaktion kann wie folgt durchgeführt werden: Ein Gemisch von Ausgangsstoff II, Lösungsmittel und Oleum wird während 0,2 bis 2 Stunden bei der Reaktionstemperatur des ersten Schrittes gehalten. Vorteilhaft wird der Harnstoff II zuerst in einem Lösungsmittel suspendiert und in das Gemisch unter guter Durchmischung das Oleum eingegeben. Dann wird das Gemisch im zweiten Reaktionsschritt während 0,2 bis 5 Stunden bei der Reaktionstemperatur des zweiten Reaktionsschrittes gehalten. Nun wird aus dem Reaktionsgemisch der Endstoff in üblicher Weise, z. B. durch Filtration, abgetrennt. Für eine grosstechnische Weiterverarbeitung des Endstoffes I ist die Abtrennung vom Lösungsmittel nicht zwingend erforderlich. Besonders vorteilhaft ist dies bei Verwendung stöchiometrischer Mengen der Reaktionskomponenten, da das Lösungsmittel nach erfolgter Reaktion nur geringe Mengen von Verunreinigung enthält.

Die nach dem Verfahren der Erfindung herstellbaren Verbindungen, sind wertvolle Ausgangsstoffe für die Herstellung von Farbstoffen und Schädlingsbekämpfungsmitteln. Beispielsweise können durch Chlorierung, z. B. mit Thionylchlorid oder Phosphorpentachlorid, die entsprechenden Amidosulfonsäurechloride, z. B. Methylamino- oder Isopropylaminosulfonylchlorid, hergestellt werden; aus ihnen kann man durch Umsetzung mit Anthranilsäure oder ihren Salzen die in der deutschen Auslegeschrift 2 104 682 beschriebenen o-Sulfamidobenzoesäuren herstellen. Durch Cyclisierung dieser Stoffe, z. B. nach dem in der deutschen Offenlegungsschrift 2 105 687 beschriebenen Verfahren, gelangt man zu den 2,1,3-Benzothiadiazin-4-on-2,2-dioxiden, deren Verwendung für Pflanzenschutzmittel und Pharmazeutika in derselben Patentschrift beschrieben ist. Bezüglich der Verwendung wird auf die vorgenannten Veröffentlichungen und auf DAS 1 120 456, DP 1 242 627 und DOS 1 542 836 verwiesen. Die aus Alkylamidosulfonsäuren herstellbaren Alkylamidosulfonylchloride sind wertvolle Ausgangsstoffe für die Synthese von den herbiciden O-(Alkylaminosulfonyl)-glykolsäureamiden (US-PS 3 883 509) durch Umsetzung mit einem substituierten Glykolsäureamid in Gegenwart eines Säureakzeptors.

Die in den folgenden Beispielen aufgeführten Teile bedeuten Gewichtsteile. Sie verhalten sich zu den Volumenteilen wie Kilogramm zu Liter.

## Beispiel 1

Methylamidosulfonsäure:

In 500 Volumenteilen 1,2-Dichloräthan werden 63 Teile N,N'-Dimethylharnstoff aufgeschlämmt. In das Gemisch werden bei 25 °C 96 Teile Oleum (65 Gew.-% $SO_3$) gegeben, dann anschliessend 36 Teile Schwefelsäure (100 Gew.-%) zugefügt. Die Reaktionszeit beträgt im ersten Schritt insgesamt 30 Minuten. Das Gemisch wird nun auf 70 bis 80 °C am Rückfluss erhitzt, wobei unter $CO_2$-Entwicklung Methylamidosulfonsäure kristallin ausfällt. Die Reaktionszeit beträgt im zweiten Schritt insgesamt 60 Minuten. Nach dem Abkühlen wird das Gemisch abgesaugt und mit 100 Volumenteilen Dichloräthan nachgewaschen.

Ausbeute: 162 Teile (praktisch quantitativ) Methylamidosulfonsäure vom Fp 182,3 °C.

## Beispiel 2

Methylamidosulfonsäure:

In 1500 Volumenteilen 1,2-Dichloräthan werden 224 Teile N,N'-Dimethylharnstoff aufgeschlämmt. In das Gemisch werden bei 25 °C 454 Teile 45gewichtsprozentiges Oleum gegeben. Das Gemisch wird 0,5 Stunden bei dieser Temperatur gerührt. Die Reaktionszeit beträgt im ersten Schritt insgesamt 45 Minuten. Danach wird das Gemisch auf 70 bis 80 °C (Rückfluss) erhitzt, wobei unter $CO_2$-Entwicklung Methylamidosulfonsäure kristallin ausfällt. Die Reaktionszeit beträgt im zweiten Schritt insgesamt 60 Minuten. Nach dem Abkühlen wird das Gemisch abgesaugt und mit Dichloräthan gewaschen.

Ausbeute: 565 Teile (praktisch quantitativ) Methylamidosulfonsäure vom Fp 182,3 °C.

## Beispiel 3

Äthylamidosulfonsäure:

In 1500 Volumenteilen 1,2-Dichloräthan werden 260 Teile N,N'-Diäthylharnstoff aufgeschlämmt. In das Gemisch werden bei 25 °C 454 Teile 45gewichtsprozentiges Oleum gegeben. Das Gemisch wird 0,5 Stunden bei dieser Temperatur gerührt. Die Reaktionszeit beträgt im ersten Schritt insgesamt 45 Minuten. Danach wird das Gemisch auf 70 °C (Rückfluss) erhitzt, wobei unter $CO_2$-Entwicklung Äthylamidosulfonsäure kristallin ausfällt. Die Reaktionszeit beträgt im zweiten Schritt insgesamt 60 Minuten. Nach dem Abkühlen auf Raumtemperatur wird das Gemisch abgesaugt und mit Dichloräthan gewaschen.

Ausbeute: 630 Teile (praktisch quantitativ) Äthylamidosulfonsäure vom Fp 170 °C (Zers.).

Beispiel 4

Isopropylamidosulfonsäure:

In 500 Volumenteilen 1,2-Dichloräthan werden 104 Teile N,N'-Diisopropylharnstoff aufgeschlämmt. In das Gemisch werden bei 0 bis 5 °C 96 Teile 65gewichtsprozentiges Oleum gegeben. Das Gemisch wird 0,5 Stunden bei 5 bis 10 °C gerührt, anschliessend werden 39 Teile 100gewichtsprozentige Schwefelsäure bei 25 °C zugegeben. Die Reaktionszeit beträgt im ersten Schritt insgesamt 45 Minuten. Das Gemisch wird unter Rückfluss (80 °C) erhitzt, bis die $CO_2$-Entwicklung beendet ist. Die Reaktionszeit beträgt im zweiten Schritt insgesamt 120 Minuten. Nach Abkühlen wird das Gemisch abgesaugt und das Filtrat mit Dichloräthan gewaschen.

Ausbeute: 195 Teile (praktisch quantitativ) Isopropylamidosulfonsäure vom Fp 167 °C.

Beispiel 5

Isopropylamidosulfonsäure:

In 500 Volumenteilen 1,2-Dichloräthan werden 104 Teile N,N'-Diisopropylharnstoff aufgeschlämmt. In das Gemisch werden bei –10 °C 139 Teile 45gewichtsprozentiges Oleum gegeben; man hält das Gemisch eine Stunde bei –4 °C bis 0 °C. Die Reaktionszeit beträgt im ersten Schritt insgesamt 80 Minuten. Nun wird aufgeheizt, wobei $CO_2$-Entwicklung einsetzt. Das Gemisch wird unter Rückfluss (80 °C) erhitzt, bis die $CO_2$-Entwicklung beendet ist. Die Reaktionszeit beträgt im zweiten Schritt insgesamt 120 Minuten. Nach Abkühlen wird das Gemisch abgesaugt und das Filtergut mit Dichloräthan gewaschen.

Ausbeute: 191 Teile (96% der Theorie) Isopropylamidosulfonsäure vom Fp 167 °C.

**Patentanspruch**

Verfahren zur Herstellung von Amidosulfonsäuren der Formel

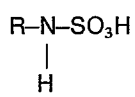 I,

worin R einen aliphatischen oder cycloaliphatischen Rest bedeutet, durch Umsetzung von Harnstoffen mit Oleum in Gegenwart organischer Lösungsmittel, dadurch gekennzeichnet, dass man substituierte Harnstoffe der Formel

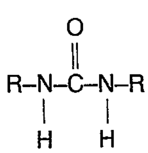 II,

worin R die vorgenannte Bedeutung hat, mit Oleum, das Schwefeltrioxid in einer Menge von 1 bis 2,5 Mol und Schwefelsäure in einer Menge von 1 bis 1,5 Mol je Mol Ausgangsstoff II enthält, in einem ersten Schritt bei einer Temperatur von –20 bis +50 °C und dann in einem zweiten Schritt bei einer Temperatur zwischen 50 und 140 °C umsetzt.

**Claim**

A process for the preparation of an amidosulfonic acid of the formula

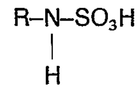 I

where R is an aliphatic or cycloaliphatic radical, by reacting a urea with oleum in the presence of an organic solvent, characterized in that a substituted urea of the formula

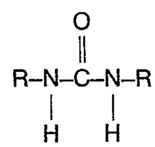 II

where R has the above meaning, is reacted with oleum, which contains from 1 to 2,5 moles of sulfur trioxide and from 1 to 1,5 moles of sulfuric acid per mole of starting material II, in a first step at from –20 to +50 °C, and then in a second step at from 50 to 140 °C.

**Revendication**

Procédé de préparation d'acides amidosulfoniques de la formule

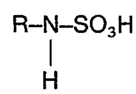 (I),

dans laquelle R représente un groupe aliphatique ou cycloaliphatique, par réaction d'urées avec l'oléum en présence de solvants organiques, caractérisé en ce que l'on fait réagir des urées substituées de la formule

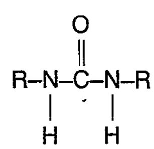 (II),

dans laquelle R possède la signification définie, avec de l'oléum contenant par mole de composé de départ II une proportion de 1 à 2,5 moles d'anhydride sulfurique et de 1 à 1,5 mole d'acide sulfurique, dans un premier stade à une température comprise entre –20 et +50 °C et ensuite dans un deuxième stade entre 50 et 140 °C.